# EUROPEAN PATENT APPLICATION

(11) **EP 4 838 540 A1**
(43) Date of publication of application: **11.11.2026**
(21) Application number: 25174810.9
(22) Date of filing: 07.05.2025
(51) Int. Cl.: G16H 40/63, G16H 40/67, G16H 15/00

(54) **METHODS AND SYSTEMS TO CUSTOMIZE ECG MONITORING**

(71) Applicant: Koninklijke Philips N.V., 5656 AE Eindhoven (NL)
(72) Inventor: LACROIX, Joyca Petra Wilma, 5656 AG Eindhoven (NL); STUT, Wilhelmus Johannes Joseph, 5656 AG Eindhoven (NL); DE JAGER, Marinus Karel Johannes, 5656 AG Eindhoven (NL)
(74) Representative: Philips Intellectual Property & Standards

(57) **Abstract**

A method (100) to customize ECG monitoring for a subject using an ECG monitoring system (200) comprising an ambulatory ECG monitor (270) and a symptom recording application (280), the method comprising: receiving (120), via a user interface (240) of the ECG monitoring system, instructions regarding symptom reporting by the subject, wherein the instructions comprise one or more parameters for implementation of the symptom recording application; generating (130) a customized symptom recording application based on the received instructions, comprising implementing the one or more parameters; generating (140), based on the received instructions, ECG monitoring instructions customized for the customized symptom recording application; and providing (150) the customized symptom recording application and the generated ECG monitoring instructions to the subject.

## Description

### FIELD OF THE INVENTION

The disclosed subject matter generally pertains to methods and symptoms for customizing ECG monitoring for a subject using an ECG monitoring system.

### BACKGROUND OF THE INVENTION

Electrocardiogram (ECG) is a non-invasive test that measures the electrical activity of the heart by placing electrodes on the chest and limbs. It is a vital diagnostic tool in cardiology, used to identify a wide range of cardiac conditions, including heart attack, arrhythmia, and heart failure. ECG can also be used to monitor the effectiveness of treatment and detect early signs of complications.

Ambulatory ECG monitors collect ECG data outside the hospital setting (e.g., in the patient's home environment or on-the-go). One of the applications of ambulatory ECG, for example, is in the diagnosis and assessment of cardiac arrhythmias or conduction abnormalities, for both symptomatic and asymptomatic patients. Typically, an ECG sensor is applied to the patient's chest, and when they feel a symptom such as chest pain or shortness of breath, they can use a computer application ("app") to describe the symptoms and the activity they were engaged in when the symptoms occurred. The user may also be able to interact with the ECG device via a sensor that allows the user to activate the sensor (such as by pressing or tapping the sensor) when they feel a symptom. The ECG data and symptom recordings are shared with the referring physician via an ECG report provided by the vendor.

While accurately-reported symptoms can be valuable when a physician is making a diagnosis or selecting a treatment, there are costs and challenges related to symptom reporting. First, it imposes a significant additional burden on the patient. Second, it imposes an additional burden on physician practices, and can burden call centers as it leads to extra calls from patients who are uncertain whether their reported symptom was properly transmitted, and from patients that have questions about the symptom reporting. Third, specifically for the interactive functionality on the sensor, some patients have difficulty doing this correctly or accidentally activate the sensor when there is no symptom. Moreover, for some patients, the reported symptoms shown in the ECG report are difficult to interpret or link to arrhythmias by the physician; this may result in a missed diagnosis or in overtreatment.

Due to the potential burden and challenges related to symptom reporting and interpretation, physicians can decide to instruct the patient to not report symptoms during the monitoring period, or to only report certain symptoms, among other options. This decision can be influenced by several factors including whether the patient has experienced symptoms prior to the monitoring, whether the physician considers the patient to be capable of symptom reporting (e.g., it may be considered too complicated or otherwise unnecessary), demographics related to the likelihood of symptoms (e.g., a younger vs. an older patient), and other situational factors related to the patient or physician.

### SUMMARY OF THE INVENTION

Thus, there is a continued need to customize ECG monitoring and symptom reporting to include or exclude symptom recording, and/or to otherwise customize the ECG monitoring as prescribed by a healthcare professional. This results in significant improvements in ECG monitoring of a subject, including analysis of the ECG monitoring by the healthcare professional.

Various embodiments and implementations are directed to methods and systems for customizing ECG monitoring for a subject using an ECG monitoring system. The system receives instructions regarding symptom reporting by the subject, comprising parameter(s) for implementation of a symptom recording application of the ECG monitoring system. A customized symptom recording application is then generated based on the received instructions, comprising implementing the one or more parameters. The system also uses the received instructions to generate ECG monitoring instructions customized for the customized symptom recording application. The customized symptom recording application and the generated ECG monitoring instructions are provided to the subject.

Notably, the application of the ECG monitoring system can be referred to as a symptom recording application, a symptom recording application, or any other symptom-related terminology. According to one embodiment, one important function of the app is to enable the user to record/report symptoms experienced while undergoing ECG monitoring. Among many other symptoms, these include heart flutters, palpitations, chest pain, dizziness, shortness of breath, fatigue, syncope, lightheadedness, sweating, and many more.

According to an aspect, a method to customize ECG monitoring for a subject using an ECG monitoring system comprising an ambulatory ECG monitor and a symptom recording application is provided. The method includes: (i) receiving, via a user interface of the ECG monitoring system, instructions regarding symptom reporting by the subject, wherein the instructions comprise one or more parameters for implementation of the symptom recording application; (ii) generating a customized symptom recording application based on the received instructions, comprising implementing the one or more parameters; (iii) generating, based on the received instructions, ECG monitoring instructions customized for the customized symptom recording application; and (iv) providing the customized symptom recording application and the generated ECG monitoring instructions to the subject.

In accordance with an embodiment, the one or more parameters comprise instructions that the subject not record any symptoms during ECG monitoring.

In accordance with an embodiment, the one or more parameters comprise instructions that the subject only record one or more predetermined symptoms during ECG monitoring.

In accordance with an embodiment, generating a customized symptom recording application based on the received instructions comprises enabling recording, by the subject, of only one or more predetermined symptoms using the symptom recording application.

In accordance with an embodiment, the received instructions comprise one or more parameters for the ambulatory ECG monitor.

In accordance with an embodiment, the one or more parameters for the ambulatory ECG monitor comprise enabling or disabling a symptom recording sensor of the ambulatory ECG monitor.

In accordance with an embodiment, receiving instructions regarding symptom reporting by the subject comprises selecting, by an instruction provider via an instruction interface of the user interface, one or more of the predetermined parameters for implementation of the symptom recording application.

In accordance with an embodiment, the method further includes: receiving, after a first time period of ECG monitoring of the subject using the ECG monitoring system, ECG data generated by the ambulatory ECG monitor; and generating a report comprising some or all of the received ECG data.

In accordance with an embodiment, the method further includes: receiving symptom recording data from the customized symptom recording application, wherein the generated report further comprises some or all of the symptom recording data from the customized symptom recording application.

In accordance with an embodiment, the one or more parameters comprise instructions that the ECG monitoring system not provide a report of symptoms when generating the report.

In accordance with an embodiment, the one or more parameters comprise instructions that predetermined non-ECG information be recorded during ECG monitoring.

According to a second aspect, an ECG monitoring system is provided. The ECG monitoring system includes: an ambulatory ECG monitor; a symptom recording application; a user interface configured to receive instructions regarding symptom reporting by the subject, wherein the instructions comprise one or more parameters for implementation of the symptom recording application; and a processor. The processor is configured to: (i) receive the instructions regarding symptom reporting by the subject; (ii) generate a customized symptom recording application based on the received instructions, comprising implementing the one or more parameters; (iii) generate, based on the received instructions, ECG monitoring instructions customized for the customized symptom recording application; and (iv) provide the customized symptom recording application and the generated ECG monitoring instructions to the subject.

In accordance with an embodiment, the one or more parameters comprise one or more of: (i) instructions that the subject not record any symptoms during ECG monitoring; (ii) instructions that the subject only record one or more predetermined symptoms during ECG monitoring; (iii) instructions enabling or disabling a symptom recording sensor (272) of the ambulatory ECG monitor; (iv) instructions that the ECG monitoring system not provide a report of symptoms when generating the report; and (v) instructions that predetermined non-ECG information be recorded during ECG monitoring.

In accordance with an embodiment, the processor is further configured to: receive, after a first time period of ECG monitoring of the subject using the ECG monitoring system, ECG data generated by the ambulatory ECG monitor; receive symptom recording data from the customized symptom recording application; and generate a report comprising some or all of the received ECG data and the received symptom recording data.

In accordance with an embodiment, the system further includes an instruction interface of the user interface, and receiving the instructions regarding symptom reporting by the subject comprises selecting, by an instruction provider via the instruction interface of the user interface, one or more of the predetermined parameters for implementation of the symptom recording application.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a method for customizing ECG monitoring, in accordance with an embodiment.
FIG. 2 is a schematic representation of an ECG monitoring system, in accordance with an embodiment.
FIG. 3 is a schematic representation of an ECG monitoring environment, in accordance with an embodiment.

### DETAILED DESCRIPTION OF EMBODIMENTS

The present disclosure describes various embodiments of a system and method configured for customizing an ECG monitoring system. More generally, Applicant has recognized and appreciated that it would be beneficial to provide methods and systems for improved ECG monitoring of a subject. The ECG monitoring system receives instructions regarding symptom reporting by the subject, comprising parameter(s) for implementation of the symptom recording application. A customized symptom recording application is then generated based on the received instructions, comprising implementing the one or more parameters. The system also uses the received instructions to generate ECG monitoring instructions customized for the customized symptom recording application. The customized symptom recording application and the generated ECG monitoring instructions are provided to the subject.

The methods and systems described or otherwise envisioned herein provide numerous advantages over existing ambulatory ECG monitoring systems and methods. For example, the systems and methods for customizing ECG monitoring overcome at least the following issues associated with the inability to customize. (1) Simplifies unnecessary complicated patient applications that can include symptom reporting functionality that the patient should not use. This is particularly problematic because a substantial portion of the target demographic for ambulatory ECG monitoring may have minimal digital skills. (2) Prevents the generation of an ECG report that includes an irrelevant section for symptoms that will be empty for patients who are requested to not report symptoms, or might include symptoms if the patient decides to report symptoms even if instructed not to do so. (3) Prevents interactive functionality of/on the symptom reporting sensor, which can result in artifacts in the ECG data and accidental interaction (e.g., erroneous capturing of a time-stamped symptom). (4) Avoids confusion on the patient side resulting from conflicting information from the physician and from ECG manuals explaining how to report symptoms.

According to an embodiment, the systems and methods described or otherwise envisioned herein can, in some non-limiting embodiments, be implemented as a component of or an extension of ECG systems. For example, one application of the embodiments and implementations herein is to improve systems such as, e.g., the Philips^{®} ECG and/or IntelliSpace^{®} line of diagnostic and reporting tools (manufactured by Koninklijke Philips, N.V.), among many other products. However, the disclosure is not limited to these devices or systems, and thus the disclosure and embodiments disclosed herein can encompass any ECG method, device, or system.

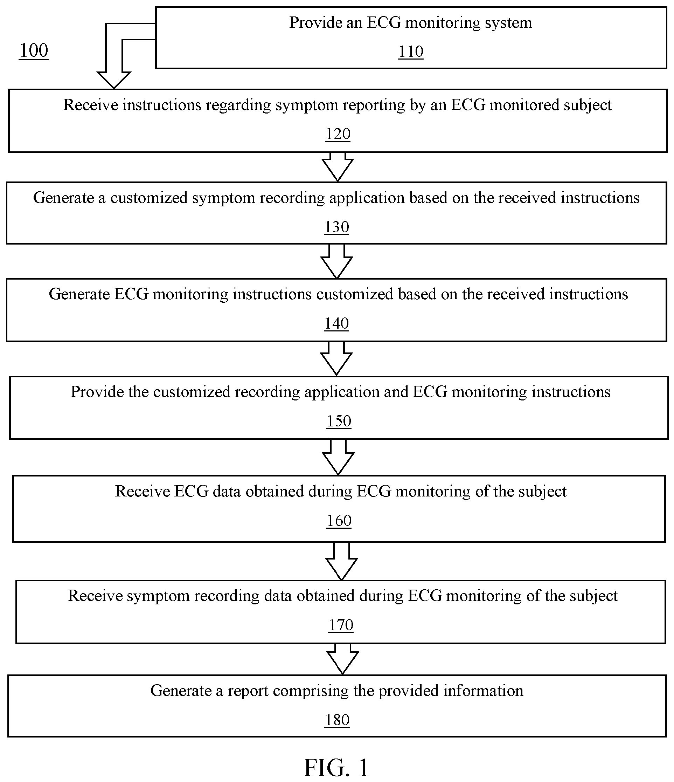

Referring to FIG. 1, in one embodiment, is a flowchart of a method 100 for customizing ECG monitoring for a subject using an ECG monitoring system. The methods described in connection with the figures are provided as examples only, and shall be understood to not limit the scope of the disclosure. The ECG monitoring system can be any of the systems described or otherwise envisioned herein. The ECG monitoring system can be a single system or multiple different systems.

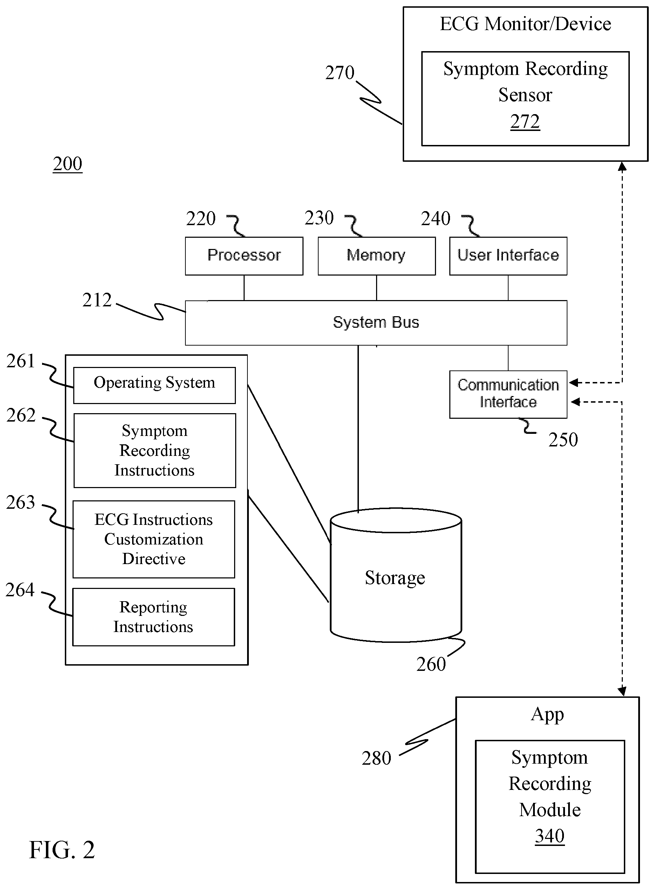

At step 110 of the method, an ECG monitoring system 200 is provided. Referring to an embodiment of an ECG monitoring system 200 as depicted in FIG. 2, for example, the system comprises one or more of a processor 220, memory 230, user interface 240, communication interface 250, and storage 260, interconnected via one or more system buses 212. It will be understood that FIG. 2 constitutes, in some respects, an abstraction and that the actual organization of the components of the system 200 may be different and more complex than illustrated. Additionally, ECG monitoring system 200 can be any of the systems described or otherwise envisioned herein. Other elements and components of ECG monitoring system 200 are disclosed and/or envisioned elsewhere herein.

According to an embodiment, the ECG monitoring system 200 is or comprises or is in local and/or remote communication with an ECG monitor or device 270. The ECG monitor or device 270 can be any ECG monitor or device, including but not limited to an ambulatory ECG monitor or device. An ambulatory ECG monitor or device is typically worn by a patient or subject for a period of time (hours, days, or weeks) as the patient or subject goes about their daily life, typically at home and work. Once the monitoring time period is complete, the patient or subject can provide the recorded ECG information to a healthcare professional. For example, the ECG monitor or device can upload the recorded ECG information (in real-time or in batches or all at once), or the patient or subject can return the ambulatory ECG monitor or device to the healthcare professional for download of the recorded ECG information. Notably, however, the methods and systems described or otherwise envisioned herein are not limited to ambulatory ECG monitors or devices.

According to an embodiment, the ECG monitor or device 270-such as a Holter monitor or a patch-based monitor-continuously captures the electrical activity of the subject's heart over an extended period, often 24 hours or more. While the subject wears the monitor, which is typically lightweight and designed for everyday activities, the subject also has access to an application, such as a smartphone app. For certain subjects, the app allows one to record details about symptoms and activities throughout the day. For example, whenever the subject experiences an unusual symptom, such as palpitations (a sudden sensation of a rapid or irregular heartbeat), chest pain or discomfort, dizziness, shortness of breath, or even episodes of sweating or anxiety, the subject can open the app and mark the event in real time. The app typically includes fields where the subject can add notes about the intensity and nature of the symptom. The healthcare provider will later attempt to correlate these reports with the ECG recordings to identify any patterns or triggers associated with the symptoms. In addition to symptoms, the subject can also log various activities that might influence their heart rate.

According to an embodiment, the ECG monitor or device 270 includes or is in communication with a symptom recording sensor 272, which allows the user to mark or timestamp the onset or occurrence of symptoms. For example, the sensor may comprise a screen or button that allows the user to tap, press, double-tap, or otherwise interact with the sensor in order to indicate the onset or occurrence of symptoms. The user can then use that timestamp to record or otherwise associate experienced symptom(s) with the timestamp.

Notably, however, as described or otherwise envisioned herein, the subject being monitored may be instructed not to record symptoms, or to only record certain symptoms. Thus, the prescribing healthcare professional (where "healthcare professional" can be anyone in the medical or healthcare field, including but not limited to physicians, clinicians, and others) may determine that the subject should not record symptoms or should only record certain symptoms using the symptom reporting app.

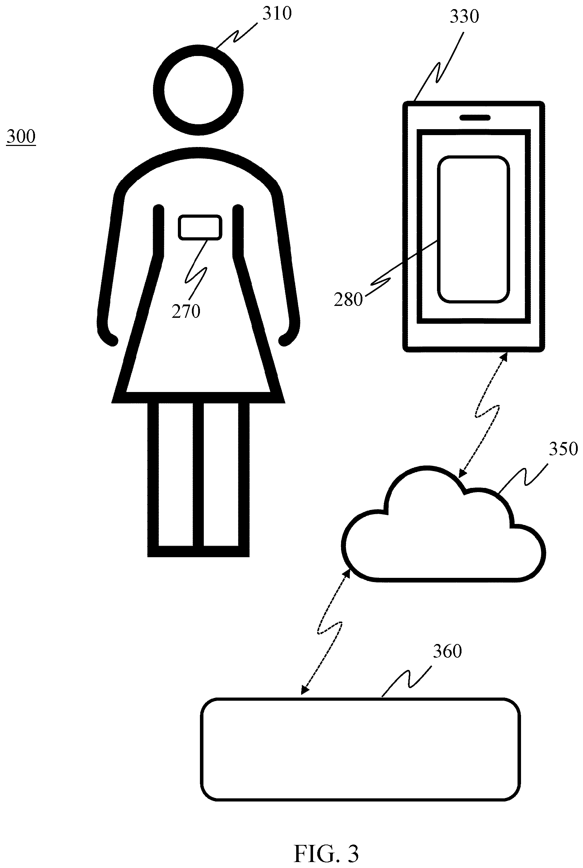

Referring to FIG. 3, in one embodiment, is an ECG monitoring environment 300. The ECG monitoring environment comprises a subject 310 that is being monitored using an ECG monitor or device 270, which may be an ambulatory ECG device. The environment further comprises a computerized device 330 with a software application ("app") 280, although the app may be hosted on any computerized device including but not limited to a smartphone, desktop, laptop, tablet, wearable, smart speaker, smart mirror, and many other possible devices.

According to an embodiment, the subject 310 feels a symptom - such as palpitation, chest pain or discomfort, dizziness, shortness of breath, sweating, or anxiety - and desires to record that symptom and optionally the activity the subject was engaged in when the symptom started, in the app 280 with symptom recording module 340. Accordingly, the subject activates or triggers or opens the symptom recording module 340 - and/or interacts with the symptom recording sensor 272 if there is one - and records one or more of the time the symptoms started, the symptoms (of which there may be one or more), and the activities that the subject was engaged in (of which there may be one or more). The app stores this information. The computerized device 330 and/or app 280 can transmit the stored information via a wired and/or wireless communication system 350 to a remote component 360 of the ECG monitoring system, such as a database or another application. This transmission of information can be in real-time, in batches, and/or all at once such as when monitoring is complete or the subject or healthcare professional directs or requests the transmission of the information.

Returning to method 100 in FIG. 1, at step 120 of the method, the ECG monitoring system 200 receives instructions regarding symptom reporting by the subject during the ECG monitoring, which can be for any period of time. The instructions can be provided by a prescribing healthcare professional, meaning a healthcare professional in charge of determining that the subject should undergo ECG monitoring, the extend of the period of monitoring, and/or the symptoms - if any - to be reported by the subject during the monitoring, among other possible instructions. Thus, the instructions comprise one or more parameters for implementation of the symptom recording application. Many different examples of the one or more parameters for implementation of the symptom recording application will be described in detail and/or are otherwise envisioned herein. According to an embodiment, the instructions can be provided by the healthcare professional via a user interface 240 of the system, and/or via a user interface of a system in remote or local communication with ECG monitoring system 200. Once received, the instructions can be implemented immediately and/or can be stored in local or remote memory for future use.

The instructions regarding symptom reporting by the subject during the ECG monitoring, comprising one or more parameters for implementation of the symptom recording application, can include a wide variety of different instructions and parameters. For example, the instructions and parameters can include instructions and parameters to, among many other things, do one or more of the following:
- enable all symptom reporting functionality in the symptom recording application;
- disable all symptom reporting functionality in the symptom recording application;
- enable some symptom reporting functionality in the symptom recording application, including an option to determine which symptom(s) should be enabled;
- disable some symptom reporting functionality in the symptom recording application, including an option to determine which symptom(s) should be disabled;
- enable active inquiry or interrogation, by the application, of the subject when the ECG system is triggered, such as when the system detects an arrhythmia or other detectable phenomenon;
- enable active inquiry or interrogation, by the application, of the subject;
- include some or all reported symptoms in an ECG report generated by the system and provided to the prescriber or another healthcare professional, including an option to determine which reported symptom(s) should be included;
- exclude some or all reported symptoms in an ECG report generated by the system and provided to the prescriber or another healthcare professional, including an option to determine which reported symptom(s) should be excluded;
- enable functionality of the symptom recording sensor 272;
- disable functionality of the symptom recording sensor 272;
- include instructions, to the subject, to report some or all symptoms during the ECG monitoring, including an option to determine which symptom(s) should be reported, where the instructions may be provided in a patient education guide, via the symptom recording application, or via an instruction website, among other locations;
- exclude instructions, to the subject, to report some or all symptoms during the ECG monitoring;
- include a physical symptom recording form (e.g., a symptom recording diary) in the supplies provided to the subject being monitored;
- exclude a physical symptom recording form (e.g., a symptom recording diary) from the supplies provided to the subject being monitored;
- modify a physical symptom recording form (e.g., a symptom recording diary) in the supplies provided to the subject being monitored, wherein the modification may add or remove symptoms to be reported by the subject; and/or many, many others.

According to an embodiment, the instructions area provided by the healthcare professional via a user interface 240 of the system, and/or via a user interface of a system in remote or local communication with ECG monitoring system 200. For example, while prescribing ECG monitoring, the healthcare professional can provide or select any of the options described or otherwise envisioned herein. The provision or selection can occur via any mechanism for providing or selecting information via a user interface. For example, among other options, the healthcare professional can use a pull-down menu, click options, touch a screen to select options, enter text, and/or use any other mechanism. Once received, the instructions can be implemented immediately and/or can be stored in local or remote memory for future use.

At step 130 of the method, the ECG monitoring system or another system generates a customized symptom recording application based on the received instructions. According to an embodiment, generating the customized symptom recording application comprises implementing the one or more parameters found in the instructions received by the system from the prescriber or other healthcare professional. Implementing the one or more parameters found in the instructions to generate a customized symptom recording application can include any action that adjusts or modifies or otherwise customizes the symptom recording application. For example, implementing the one or more parameters can include turning options on or off, altering the programming of the symptom recording application, changing the functionality of the symptom recording sensor 272 or any other component of the ECG monitoring system, or any other action.

According to an embodiment, the one or more parameters can enable the ECG monitoring system to be used to report only a specific set of symptoms when they occur (and to not report any other symptoms). For example, at the moment of prescription of the ECG service to a patient, the healthcare professional can enter whether they wants the patient to report all possible symptoms (full symptom reporting), a particular subset of symptoms (partial symptom reporting), or no symptoms (no symptom reporting). Among many other mechanisms, this can be accomplished by checking the corresponding boxes in the interface: (i) Checkbox: "do not include symptom reporting", (ii) Checkbox "include monitoring of all symptoms"; (iii) Checkbox: "include monitoring of the following symptoms only": (1) Checkbox: Short breath; (2) Checkbox: Heart racing; (3) Checkbox: Light-headed; (4) Checkbox: etc.

According to an embodiment, when an account is created for a subject that will be ECG monitored, the account or subject profile contains information about whether the patient is prescribed to report symptoms during the monitoring period (and optionally which symptoms). Thus, for example, if a healthcare professional prescribes ECG monitoring without symptom recording, when logging into the symptom recording application with the patient account credentials, the application disables all symptom reporting functionality in line with the information in the patient profile. Thus, for example, symptom reporting will be disabled and the application will not actively inquire about symptoms when an arrhythmia or other potentially triggering event is detected.

According to an embodiment, when an account is created for a subject that will be ECG monitored, the account or subject profile contains information about whether the patient is prescribed to report symptoms during the monitoring period (and optionally which symptoms). Thus, for example, if a healthcare professional prescribes ECG monitoring with symptom recording (e.g., all symptoms or some symptom(s)), when logging into the symptom recording application with the patient account credentials, the application enables or activates (or fails to disable) some or all symptom reporting functionality in line with information in the patient profile. Thus, for example, symptom reporting will be enabled (for only the symptom(s) prescribed by the healthcare professional to be reported), and the application can actively inquire about symptoms when an arrhythmia or other potentially triggering event is detected.

At step 140 of the method, the ECG monitoring system or another system generates customized ECG monitoring instructions based on the received instructions. These ECG monitoring instructions are intended for the subject to read prior to ECG monitoring and to follow during the ECG monitoring. According to an embodiment, the ECG monitoring instructions are customized based on the received instructions.

The ECG monitoring instructions can be provided to the subject in a wide variety of ways, each of which may affect how the ECG monitoring instructions are customized. According to an embodiment, the ECG monitoring instructions are provided to the subject via a patient education guide, via the symptom recording application, or via an instruction website, among other locations. The ECG monitoring instructions can be mailed to the subject (such as with components of the ECG monitoring system such as the ambulatory ECG sensor), provided to the subject at a healthcare facility, or retrieved by the subject online or digitally, such as using a QR code or other pointer or digital source.

According to an embodiment, when an account is created for a subject that will be ECG monitored, the account or subject profile contains information about whether the patient is prescribed to report symptoms during the monitoring period (and optionally which symptoms). Thus, for example, if a healthcare professional prescribes ECG monitoring without symptom recording, then no physical symptom recording form (e.g., a symptom recording diary) will be provided to the subject. As another example, physical ECG monitoring instructions provided to the subject may comprise educational materials in line with the patient's profile (i.e., excluding the symptom reporting explanations and app visuals). Similarly, the ECG monitoring system may comprise a QR code or other pointer (such as a web address) that links to digital ECG monitoring instructions that comprise the educational materials in line with the patient's profile.

As another example, if a healthcare professional prescribes ECG monitoring with symptom recording (e.g., all symptoms or some symptom(s)), then a physical symptom recording form (e.g., a symptom recording diary) can be provided to the subject. As another example, physical ECG monitoring instructions provided to the subject may comprise educational materials in line with the patient's profile (i.e., including the symptom reporting explanations and app visuals, optionally including instructions for reporting only a specific predetermined subset of possible symptoms). As another example, physical ECG monitoring instructions provided to the subject may comprise educational materials in line with the patient's profile (i.e., including the symptom reporting explanations and app visuals). Similarly, the ECG monitoring system may comprise a QR code or other pointer (such as a web address) that links to digital ECG monitoring instructions that comprise the educational materials in line with the patient's profile.

At step 150 of the method, the customized symptom recording application and the generated ECG monitoring instructions are provided to the subject. Notably, the customized symptom recording application and the generated ECG monitoring instructions can be provided to the subject with the ECG monitor or device 270, or separate from the ECG monitor or device 270. For example, the subject may receive a mailed package that contains the ECG monitor or device 270, physical copy of the ECG monitoring instructions, and/or directions to access the customized symptom recording application and/or generated ECG monitoring instructions. As another example, the subject may pick up the ECG monitor or device 270, physical copy of the ECG monitoring instructions, and/or directions to access the customized symptom recording application and/or generated ECG monitoring instructions at a healthcare facility. As another example, the subject may receive the ECG monitor or device 270 via the mail or via pickup or delivery, and may then receive a physical copy of the ECG monitoring instructions and/or directions to access the customized symptom recording application and/or generated ECG monitoring instructions separately from the ECG monitor or device 270. For example, the subject may receive directions to access the customized symptom recording application and/or generated ECG monitoring instructions via email or another delivery service.

At step 160 of the method, the ECG monitoring system receives, after a first time period of ECG monitoring of the subject using the ECG monitoring system, ECG data generated by the ambulatory ECG monitor. Similarly, at step 170 of the method, the ECG monitoring system receives, after a first time period of ECG monitoring of the subject using the ECG monitoring system, symptom data from the customized symptom recording application. Of course, the symptom data is dependent upon the prescriber's decisions and may be information about recorded symptoms or only an instruction that no symptoms were to be recorded. Steps 160 and 170 may occur simultaneously and together, or separately and at different times.

According to an embodiment, therefore, the ambulatory ECG monitor or device 270 can record ECG data for the subject during the prescribed first time period. The system can also record and provide - or not record or provide - symptoms experienced by the subject and recorded (or not recorded) via the customized symptom recording application. This symptom recording data can also be provided to one or more other components of the ECG monitoring system, simultaneously and together with, or separately from, the ECG data provided to one or more other components of the ECG monitoring system. For example, the subject may mail or deliver the ambulatory ECG monitor or device 270 to the healthcare professional or setting at the end of the first time period. This may include a transfer of the ECG data and/or symptom recording data, or the ECG data and symptom recording data may be provided to or requested by one or more other components of the ECG monitoring system. This provision or request may be triggered by the healthcare professional, by the subject, or otherwise triggered.

At step 180 of the method, report comprising some or all of the received ECG data and/or the received symptom recording data. The report may comprise any of the information described or otherwise envisioned herein. The system may provide the report on a display, such as an ECG user interface or any other user interface. According to an embodiment, the provided output can be any of the information as described or otherwise envisioned herein. The system may provide the information to a user via any mechanism, including but not limited to a visual display. The information may be communicated by wired and/or wireless communication to another device. For example, the system may communicate the information to a monitor, screen, mobile phone, computer, laptop, wearable device, and/or any other device configured to allow display and/or other communication of the information.

According to an embodiment of the methods and systems described or otherwise envisioned herein, the one or more parameters found in the instructions regarding symptom reporting by the subject during the ECG monitoring can comprise instructions that predetermined non-ECG information be recorded during ECG monitoring. The non-ECG information can be recorded, for example, using the customized symptom recording application. The non-ECG information can include any non-ECG information, such as blood pressure measurements and/or medication intake, among others. Healthcare professionals would like to have blood pressure measurements (at selected moments or continuously) obtained during an ECG monitoring period, in order to help them make the proper diagnosis and determine treatment. Also, for some patients, healthcare professionals need to know if the patient took medication close in time to an experienced symptom to help them interpret the symptom and the ECG signal. Thus, just as the symptom recording application and/or ECG monitoring instructions can be customized based on whether and/or which symptoms to report or record, the symptom recording application and/or ECG monitoring instructions can be customized pursuant to the healthcare professional's instructions to report blood pressure measurements and/or medication intake. This would lead to a service and healthcare professional report that contains the relevant functionality and information both for the subject and healthcare professional.

Referring again to FIG. 2 is a schematic representation of an ECG monitoring system 200. System 200 may be any of the devices or systems described or otherwise envisioned herein, and may comprise any of the components described or otherwise envisioned herein. It will be understood that FIG. 2 constitutes, in some respects, an abstraction and that the actual organization of the components of system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. **In** some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. **In** various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. **In** this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, storage 260 of system 200 may store one or more algorithms, modules, and/or instructions to carry out one or more functions or steps of the methods described or otherwise envisioned herein. For example, storage 260 may comprise, among other instructions or data, symptom recording instructions 262, ECG instructions customization directive 263, and/or reporting instructions 264.

According to an embodiment, symptom recording instructions 262 direct the system to customize the symptom recording application based on the instructions received from the prescriber. According to an embodiment, generating the customized symptom recording application comprises implementing the one or more parameters found in the instructions received by the system from the prescriber or other healthcare professional. Implementing the one or more parameters found in the instructions to generate a customized symptom recording application can include any action that adjusts or modifies or otherwise customizes the symptom recording application. For example, implementing the one or more parameters can include turning options on or off, altering the programming of the symptom recording application, changing the functionality of the symptom recording sensor 272 or any other component of the ECG monitoring system, or any other action. Thus, the symptom recording instructions 262 comprise programming, instructions, directions, or other mechanisms to generate the customized symptom recording application by implementing the one or more parameters found in the instructions received by the system from the prescriber or other healthcare professional. Once generated, the customized symptom recording application can be utilized immediately (such as providing the application to the subject or otherwise making it available to the subject), or it can be stored in local and/or remote memory for future use.

According to an embodiment, ECG instructions customization directive 263 directs the system to generate customized ECG monitoring instructions based on the instructions received from the prescriber. These ECG monitoring instructions are intended for the subject to read prior to ECG monitoring and to follow during the ECG monitoring. According to an embodiment, generating the customized ECG monitoring instructions comprises implementing the one or more parameters found in the instructions received by the system from the prescriber or other healthcare professional. Implementing the one or more parameters found in the instructions to generate a customized ECG monitoring instructions can include any action that adjusts or modifies or otherwise customizes the ECG monitoring instructions. For example, implementing the one or more parameters can include providing or not providing information in the ECG monitoring instructions, among other implementations. Thus, the ECG instructions customization directive 262 comprises programming, instructions, directions, or other mechanisms to generate the customized ECG instructions by implementing the one or more parameters found in the instructions received by the system from the prescriber or other healthcare professional. Once generated, the customized ECG instructions can be utilized immediately (such as providing the ECG instructions to the subject or otherwise making them available to the subject), or they can be stored in local and/or remote memory for future use.

According to an embodiment, reporting instructions 264 direct the system to generate and provide a report to a healthcare professional, subject, and/or to another device or system. The report can comprise any of the information as described or otherwise envisioned herein, including but not limited to the received ECG data and/or the received symptom recording data. These received data can be collated and provided on one display or screen for ease of interpretation, or may be provided separately. The system may provide the information to a user via any mechanism, including but not limited to a visual display. The information may be communicated by wired and/or wireless communication to another device. For example, the system may communicate the information to a monitor, screen, mobile phone, computer, laptop, wearable device, and/or any other device configured to allow display and/or other communication of the information.

It should be appreciated that all combinations of the foregoing concepts and additional concepts discussed in greater detail herein (provided such concepts are not mutually inconsistent) are contemplated as being part of the inventive subject matter disclosed herein. **In** particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) to customize ECG monitoring for a subject using an ECG monitoring system (200) comprising an ambulatory ECG monitor (270) and a symptom recording application (280), the method comprising:
receiving (120), via a user interface (240) of the ECG monitoring system, instructions regarding symptom reporting by the subject, wherein the instructions comprise one or more parameters for implementation of the symptom recording application;
generating (130) a customized symptom recording application based on the received instructions, comprising implementing the one or more parameters;
generating (140), based on the received instructions, ECG monitoring instructions customized for the customized symptom recording application; and
providing (150) the customized symptom recording application and the generated ECG monitoring instructions to the subject.

2. The method of claim 1, wherein the one or more parameters comprise instructions that the subject not record any symptoms during ECG monitoring.

3. The method of claim 1, wherein the one or more parameters comprise instructions that the subject only record one or more predetermined symptoms during ECG monitoring.

4. The method of claim 1, wherein generating a customized symptom recording application based on the received instructions comprises enabling recording, by the subject, of only one or more predetermined symptoms using the symptom recording application.

5. The method of claim 1, wherein the received instructions comprise one or more parameters for the ambulatory ECG monitor.

6. The method of claim 5, wherein the one or more parameters for the ambulatory ECG monitor comprise enabling or disabling a symptom recording sensor (272) of the ambulatory ECG monitor.

7. The method of claim 1, wherein receiving instructions regarding symptom reporting by the subject comprises selecting, by an instruction provider via an instruction interface of the user interface, one or more of the predetermined parameters for implementation of the symptom recording application.

8. The method of claim 1, further comprising:
receiving (160), after a first time period of ECG monitoring of the subject using the ECG monitoring system, ECG data generated by the ambulatory ECG monitor; and
generating (180) a report comprising some or all of the received ECG data.

9. The method of claim 8, further comprising receiving (170) symptom recording data from the customized symptom recording application, wherein the generated report further comprises some or all of the symptom recording data from the customized symptom recording application.

10. The method of claim 8, wherein the one or more parameters comprise instructions that the ECG monitoring system not provide a report of symptoms when generating the report.

11. The method of claim 1, wherein the one or more parameters comprise instructions that predetermined non-ECG information be recorded during ECG monitoring.

12. An ECG monitoring system (200), comprising:
an ambulatory ECG monitor (270);
a symptom recording application (280);
a user interface (240) configured to receive instructions regarding symptom reporting by the subject, wherein the instructions comprise one or more parameters for implementation of the symptom recording application; and
a processor (220) configured to: (i) receive the instructions regarding symptom reporting by the subject; (ii) generate a customized symptom recording application based on the received instructions, comprising implementing the one or more parameters; (iii) generate, based on the received instructions, ECG monitoring instructions customized for the customized symptom recording application; and (iv) provide the customized symptom recording application and the generated ECG monitoring instructions to the subject.

13. The system of claim 12, wherein the one or more parameters comprise one or more of: (i) instructions that the subject not record any symptoms during ECG monitoring; (ii) instructions that the subject only record one or more predetermined symptoms during ECG monitoring; (iii) instructions enabling or disabling a symptom recording sensor (272) of the ambulatory ECG monitor; (iv) instructions that the ECG monitoring system not provide a report of symptoms when generating the report; and (v) instructions that predetermined non-ECG information be recorded during ECG monitoring.

14. The system of claim 12, wherein the processor is further configured to: receive, after a first time period of ECG monitoring of the subject using the ECG monitoring system, ECG data generated by the ambulatory ECG monitor; receive symptom recording data from the customized symptom recording application; and generate a report comprising some or all of the received ECG data and the received symptom recording data.

15. The system of claim 12, further comprising an instruction interface of the user interface, and wherein receiving the instructions regarding symptom reporting by the subject comprises selecting, by an instruction provider via the instruction interface of the user interface, one or more of the predetermined parameters for implementation of the symptom recording application.
